# EUROPÄISCHE PATENTSCHRIFT

(11) **EP 1 231 955 B1**
(45) Veröffentlichungstag und Bekanntmachung des Hinweises auf die Patenterteilung: **26.11.2003**
(21) Anmeldenummer: 00929551.0
(22) Anmeldetag: 25.05.2000
(51) Int. Cl.: A61M 1/06

(54) **MILCHABSAUGPUMPE**
MILK SUCKING PUMP
POMPE D'ASPIRATION POUR LE LAIT

(30) Priorität: 11.11.1999 DE 19954112
(43) Veröffentlichungstag der Anmeldung: 21.08.2002
(73) Patentinhaber: Kaweco GmbH, 71254 Ditzingen (DE)
(72) Erfinder: KIRCHNER, Claudia, D-71706 Markgröningen (DE)
(74) Vertreter: Fleck, Hermann-Josef, Dr.-Ing.
(86) Internationale Anmeldenummer: EP0004758
(87) Internationale Veröffentlichungsnummer: WO01034226

(56) Entgegenhaltungen:
- EP-A- 0 733 376
- DE-U- 8 714 995
- US-A- 5 542 921
- US-A- 5 843 029

## Beschreibung

Die Erfindung bezieht sich auf eine Milchabsaugpumpe mit einem an der Öffnung eines Behälters lösbar angebrachten oder anbringbaren, ein Brustansatzstück aufweisenden Aufsatzteil und einer an diesem mittels eines Anschlussstutzens oder einer Anschlussbohrung lösbar angeschlossenen Handpumpeneinheit, die einen kappenartigen Anschlussabschnitt sowie einen in einem Hubraum mittels eines schwenkbaren und mit einem Rückholmechanismus versehenen Betätigungsgriffes hin und her bewegbaren Pumpkolben aufweist.

Eine derartige Milchabsaugpumpe mit einer Handpumpeneinheit ist in der DE 87 14 995 U1 angegeben. Bei dieser bekannten Milchabsaugpumpe ist auf einen Behälter ein Aufsatzteil mit einem trichterförmigen Brustansatzstück und einem Pumpenanschluss lösbar aufgeschraubt. Auf das Pumpanschlussstück wird ein kappenförmiger Abschnitt der Handpumpeneinheit aufgesetzt. An den kappenartigen Abschnitt schließt sich nach hinten horizontal abstehend ein Pumpzylinder mit einem im Inneren geführten Pumpkolben an. Der Pumpkolben wird mittels eines hebelförmigen Betätigungsgriffes hin und her bewegt, wobei der Griff zum Rückführen des Pumpkolbens mittels einer U-förmigen Feder an einer unter dem Pumpzylinder befestigten Halterung abgestützt ist, die sich bis auf die Unterseite des Behälters erstreckt. Der Aufbau der Handpumpeneinheit ist relativ aufwendig und sperrig, so dass sich auch Nachteile bei der Handhabung ergeben können.

Die EP-A 0 733 376 zeigt eine Milchabsaugpumpe mit Aufsatzteil und daran angebrachter Handpumpeneinheit, wobei auf einem oberen Rand des nach oben offenen Aufsatzteils ein elastisches Diaphragma aufgesetzt wird, das mittels eines Betätigungsgriffes gegen eine Federkraft anhebbar und mit der Federkraft absenkbar ist. Hierdurch wird ein in dem Aufsatzteil unterhalb des Diaphragmas gebildeter Saugraum, an den das Brustansatzstück angeschlossen ist, zum Pumpen vergrößert und verkleinert. Oberhalb des Diaphragmas ist ein nach oben konvexer Deckel aufgesetzt, der an dem Aufsatzteil mittels seitlicher Rastfinger verrastet ist. Der Betätigungsgriff ist durch eine Öffnung des Deckels geführt. Die Handpumpeneinheit ist damit teilweise in den Aufsatzteil integriert und wird beim Trennen von diesem z.B. zum Reinigen in mehrere Einzelheiten zerlegt, wodurch die Handhabung ebenfalls benachteiligt ist.

Bei einer in der EP 0 330 845 A2 gezeigten weiteren Milchabsaugpumpe besitzt eine Handpumpeneinheit einen nach hinten aus dem Pumpzylinder axial herausragenden Handbetätigungsteil, der mit einer Hand in axialer Richtung bewegt wird, während mit der anderen Hand die Milchabsaugpumpe gehalten wird. Für die Handhabung werden somit beide Hände benötigt.

Bei einer in der EP 0 385 933 A2 gezeigten Milchabsaugpumpe ist ein Pumpzylinder schräg nach oben gerichtet. Ein in seinem Inneren geführter Pumpkolben wird mittels zweier seitlich an dem Pumpzylinder vorbei geführter Betätigungselemente und eines an diesen oberhalb des Pumpzylinders gelenkig befestigten Querstegs betätigt, der sich beim Zusammendrücken der Betätigungselemente in der Mitte nach oben und beim Loslassen nach unten bewegt, um den Pumpkolben hin und her zu verschieben. Die Betätigungseinheit mit dem Pumpkolben kann durch eine elektrische Pumpe ersetzt werden, die mittels eines auf die Oberseite des Pumpzylinders aufsetzbaren Deckels angeschlossen wird.

Der Erfindung liegt die Aufgabe zugrunde, eine Milchabsaugpumpe der eingangs genannten Art bereit zu stellen, die bei vereinfachtem Aufbau eine verbesserte Handhabbarkeit ergibt.

Diese Aufgabe wird mit den Merkmalen des Anspruches 1 gelöst. Hiernach ist vorgesehen, dass der kappenartige Anschlussabschnitt und der Hubraum in einem als einheitliches kappenförmiges Teil ausgebildeten Kappenteil zusammengefasst sind, das mit Haltemitteln ausschließlich an dem Aufsatzteil festgelegt ist, und dass der einerseits an dem Betätigungsgriff angreifende Rückholmechanismus andererseits an dem Kappenteil gelagert ist.

Der als einheitliches kappenförmiges Teil ausgebildete Kappenteil ergibt eine für die Handhabung und Reinigung günstige Form und ist mit den an dem Aufsatzteil festzulegenden Haltemitteln leicht anbringbar und abnehmbar, so dass auch der Behälter ohne weiteres von dem Aufsatzteil abgenommen werden kann. Zur einfachen Handhabung trägt auch die Anordnung des Rückholmechanismus an dem Betätigungsgriff und dem Kappenteil bei.

Eine einfache Reinigung und Handhabung werden dadurch unterstützt, dass eine Hubraumöffnung auf der dem Brustansatzstück abgekehrten Seite des Hubraums im vollkommen eingeführten Zustand des Pumpkolbens mit einem in Gebrauchsstellung oberhalb einer Schwenkachse liegenden oberen Abschnitt des Betätigungsgriffes abgedeckt ist.

Eine leichtgängige, auf Dauer weitgehend verschleißfreie Kolbenbewegung und der angeschlossenen Betätigungsteile wird dadurch erzielt, dass der Hubraum in dem Kappenteil entsprechend einer Bewegungsbahn des von dem oberen Abschnitt des Betätigungsgriffes bewegten Pumpkolbens bogenförmig gekrümmt ist.

Ein Eintritt von Luft in den Kolbenraum und die Pumpwirkung werden dadurch begünstigt, dass ein Schwenkweg des oberen Abschnittes des Betätigungsgriffes im Bereich seiner Anbindung an den Pumpkolben bei angebrachtem Behälter so groß gewählt ist, dass zumindest ein oberer Randabschnitt des Pumpkolbens im herausbewegten Zustand außerhalb eines oberen Öffnungsrandes der Hubraumöffnung liegt. Ähnlich könnte auch z.B. eine Nut oder eine Bohrung oder dgl. in der Kolbenwandung eingebracht sein.

Mit den Maßnahmen, dass der Rückholmechanismus mindestens eine Zugfeder aufweist und dass ein Einhängeelement an dem Betätigungsgriff und ein weiteres Einhängeelement an dem Kappenteil derart positioniert sind, dass bei eingeführter Kolbenstellung die Richtung der Zugkraft oberhalb der Schwenkachse des Betätigungsgriffes liegt, zumindest bis der Betätigungsgriff bei angebrachtem Behälter seinen maximalen Schwenkwinkel in Ausführrichtung des Pumpkolbens erreicht, und dass bei abgenommenem Behälter und weiter vergrößertem Schwenkwinkel die Richtung der Zugkraft unterhalb die Schwenkachse tritt, so dass der Betätigungsgriff gegenüber dem Kappenteil in der geöffneten Stellung gehalten ist, werden ein einfacher, gut funktionierender Rückholmechanismus geschaffen und die Reinigung begünstigt.

Eine alternative Ausgestaltung eines gut funktionierenden, einfachen Rückholmechanismus besteht darin, dass der Rückholmechanismus mindestens eine Druckfeder aufweist, dass ein Stützelement auf der Innenseite des Betätigungsgriffs und ein Stützabschnitt an dem Kappenteil derart positioniert sind, dass zumindest bei eingeführter Kolbenstange die Richtung der Druckkraft unterhalb der Schwenkachse des Betätigungsgriffes liegt. Dabei kann z.B. ein Gummiblock, eine Spiralfeder oder eine Biegefeder verwendet werden.

Eine auf Dauer stabil bleibende Rückstellkraft wird hierbei mit einfachen Maßnahmen dadurch erreicht, dass die Druckfeder als Biegefeder ausgebildet ist, die mit einer vorderen Einhängenase an einem Stützabschnitt in einem beim Gebrauch nach unten gerichteten freien Endabschnitt des Haltestückes eingehängt und abgestützt ist, sich in einen Innenraum von Kappenteil und Haltegriff U-förmig nach oben aufwölbt und mit einem von der Einhängenase abgebogenen freien Endbereich an einem auf der Innenseite des Betätigungsgriffes angeordneten Stützelement abgestützt ist. Der Stützabschnitt und das Stützelement können dabei an dem Haltestück bzw. Betätigungsgriff angeformt sein. Die z.B. aus Stahl bestehende Biegefeder ist thermisch stabil und kann durch Kochen gereinigt werden. Sie ist einfach montierbar und leicht optisch verdeckt unterbringbar.

Dadurch, dass an dem an dem Aufsatzteil angeordneten Anschlussstutzen oder der Anschlussbohrung eine Elektropumpe unmittelbar mit einem Schlauch anschließbar ist, lässt sich die Handpumpeneinheit leicht durch eine Elektropumpe ersetzen.

Eine einfache Abdichtung, die eine gute Pumpwirkung unterstützt, wird dadurch erzielt, dass die Ankoppelstelle zwischen dem Anschlussstutzen oder der Anschlussbohrung und dem Kappenteil mittels einer Konusverbindung oder einem Dichtring abgedichtet ist.

Mit den Maßnahmen, dass an dem Aufsatzteil im Bereich des Anschlussstutzens oder der Anschlussbohrung eine mittels eines Stopfens und, bei abgenommenem Stopfen, von Hand verschließbare Öffnung vorgesehen ist, kann bei Elektrobetrieb die Belüftung von Hand reguliert werden, wenn die Elektropumpe keine Automatikbelüftung besitzt.

Eine einfach lösbare Ankopplung zwischen dem Aufsatzteil und der Handpumpeneinheit wird dadurch erzielt, dass die Haltemittel ein Rastelement aufweisen, das bei an dem Aufsatzteil angekoppeltem Kappenteil an dem Aufsatzteil verrastet. Die Ausbildung und Bedienung ist dabei durch die Maßnahmen günstig, dass das Rastelement als mit einem freien Endabschnitt zu dem Behälter gerichtete Rastzunge ausgebildet ist, die im aufgesetzten Zustand mit einem Rastabsatz einen zum Behälter gekehrten Rand des Aufsatzteils hintergreift, wenn das Kappenteil der Rastzunge gegenüberliegend auf den bezüglich des Behälters achsparallel gerichteten Anschlussstutzen oder die Anschlussbohrung aufgeschoben ist.

Die Bedienung wird weiterhin dadurch erleichtert, dass der Kappenteil auf seiner Innenseite beidseitig Haltelappen oder Rippen trägt, die Führungselemente beim Aufsetzen auf das Aufsatzteil und Sicherungselemente gegen ein Verdrehen des Kappenteils bezüglich des Aufsatzteils bilden.

Weiterhin tragen zum einfachen Aufbau und zur einfachen Bedienung bei, dass an beiden Seitenabschnitten des Kappenteils einerseits und beiden Seitenabschnitten des Betätigungsgriffes andererseits Lagerelemente angeordnet sind, die als trennbare Lagerzapfen/Lagerauge-Verbindung die Schwenkachse zwischen Betätigungsgriff und Kappenteil bilden.

Eine für die Reinigung und Handhabung günstige Form der Handpumpeneinheit besteht darin, dass das Kappenteil eine gerundete Oberseite aufweist, die in dem von dem Brustansatzstück abgekehrten hinteren Bereich im eingeschwenkten Zustand des oberen Abschnittes des Betätigungsgriffes stetig in dessen ebenfalls gekrümmte Außenseite übergeht.

Die Betätigung der Handpumpe wird dadurch erleichtert, dass der obere Abschnitt und der untere Abschnitt im Querschnitt auf ihrer von dem Brustansatzstück abgekehrten Rückseite nach außen gerundet sind und stetig ineinander übergehen, wobei zwischen dem oberen Abschnitt und dem unteren Abschnitt ein nach hinten offener stumpfer Winkel gebildet ist.

Eine einfache Änderung der Pumpleistung wird dadurch erreicht, dass ein in den beim Verschwenken des Betätigungsgriffes im oberen Bereich zwischen dessen oberem Abschnitt und dem Rand der Hubraumöffnung gebildeten V-förmigen Spalt einsetzbares Zwischenstück vorgesehen ist, mit dem der Hubweg des Pumpenkolbens stufenlos oder abgestuft vorgebbar ist.

Mit den Maßnahmen, dass auf der Innenseite eines Schraubanschlusses des Aufsatzteils zum Verbinden mit dem Behälter Abstandsnocken vorgesehen sind, die im aufgesetzten Zustand mit dem oberen Rand des Behälters in Berührung treten, so dass im aufgesetzten Zustand ein Luftaustausch mit der Atmosphäre gegeben ist, wird erreicht, dass bei auf den Behälter aufgeschraubtem Aufsatzteil eine Belüftung des Behälters sichergestellt ist.

Zur Anbindung des Pumpkolbens bei einfachem Aufbau sind die Maßnahmen vorteilhaft, dass der Pumpkolben zentrisch oder exzentrisch nach oben versetzt eine angeformte Kolbenstange aufweist, an dessen nach hinten gerichtetem Endabschnitt eine lösbare, gelenkige Verbindung mit dem oberen Abschnitt des Betätigungsgriffes hergestellt ist.

Ist vorgesehen, dass auf der behälterseitigen Innenseite des unteren Abschnittes des Betätigungsgriffes ein Vorsprung aus weichem Material zum Bilden eines Anschlages zwischen dem Betätigungsgriff und dem Behälter angeordnet ist, so wird ein hartes Anschlagen des Betätigungsgriffes an der Behälterwandung vermieden. Der Anschlag kann dabei auch zur Veränderung des Schwenkweges zur Betätigung des Pumpkolbens ausgelegt sein.

Zu einer guten Handhabung tragen außerdem die Maßnahmen bei, dass die Handpumpeneinheit und das Aufsatzteil so angeordnet und in ihrem Gewicht abgeglichen sind, dass der Behälter im leeren Zustand bei aufgesetztem Aufsatzteil und angebrachter Handpumpeneinheit aufrecht stehen bleibt.

Das Innere der Milchabsaugpumpe kann variabel belüftet werden durch die Maßnahmen, dass an dem Kappenteil eine von außen manuell betätigbare Beiluftreguliereinheit zur Belüftung des bei einer Pumpenbetätigung variierten Saugraums vorgesehen ist.

Ein einfacher Aufbau mit leichter Handhabbarkeit besteht dabei darin, dass die Beiluftreguliereinheit ein mit einem außen auf dem Kappenteil angeordneten, drehbaren Einsatzteil und/oder Aufsatzteil versehen ist, mit dessen oder deren Verdrehung ein durch eine Wandung des Kappenteils in den Hubraum führender Durchtrittskanal mehr oder weniger weit geöffnet wird oder ganz verschließbar ist. Die Einstellung ist dabei vorzugsweise stufenlos und z.B. mittels fühlbarer Markierung reproduzierbar. Ein Vakuum wird bei geöffnetem Durchlasskanal automatisch abgebaut und die Höhe des Vakuums kann individuell gewählt werden. Eine Herstellung aus Silikon ist günstig für die Hygiene.

Die Erfindung wird nachfolgend anhand eines Ausführungsbeispiels unter Bezugnahme auf die Zeichnungen näher erläutert. Es zeigen:
- Fig. 1 A bis 1 E: Ansichten einer Milchabsaugpumpe mit Behälter von der Seite, von vorne, von hinten, von oben bzw. in perspektivischer Darstellung,
- Fig. 2A bis 2E: eine bei der Milchabsaugpumpe verwendete Handpumpeneinheit in seitlicher Ansicht, von hinten, von vorne, von oben bzw. in perspektivischer Ansicht,
- Fig. 3A bis 3F: einen bei der Milchabsaugpumpe verwendeten Betätigungsgriff von vorne, von der rechten Seite, von der linken Seite, von hinten, von unten bzw. von oben,
- Fig. 4A bis 4E: einen bei der Milchabsaugpumpe verwendeten Kappenteil von der Seite, von hinten, von vorne, von unten, von oben bzw. in perspektivischer Ansicht,
- Fig. 5A bis 5E: einen bei der Milchabsaugpumpe verwendeten Pumpkolben in perspektivischer Ansicht, von oben, von der Seite, von hinten bzw. von vorne,
- Fig. 6A bis 6E: eine weitere Handpumpeneinheit, bei der gegenüber den vorangehenden Ausführungsbeispielen ein anderer Rückholmechanismus und eine Beiluftreguliereinheit vorgesehen sind, und
- Fig. 7A und 7B: einen Querschnitt der Handpumpeneinheit nach Fig. 6A, 6D und 6E in einer Ansicht von unten bzw. einer Detailansicht x.

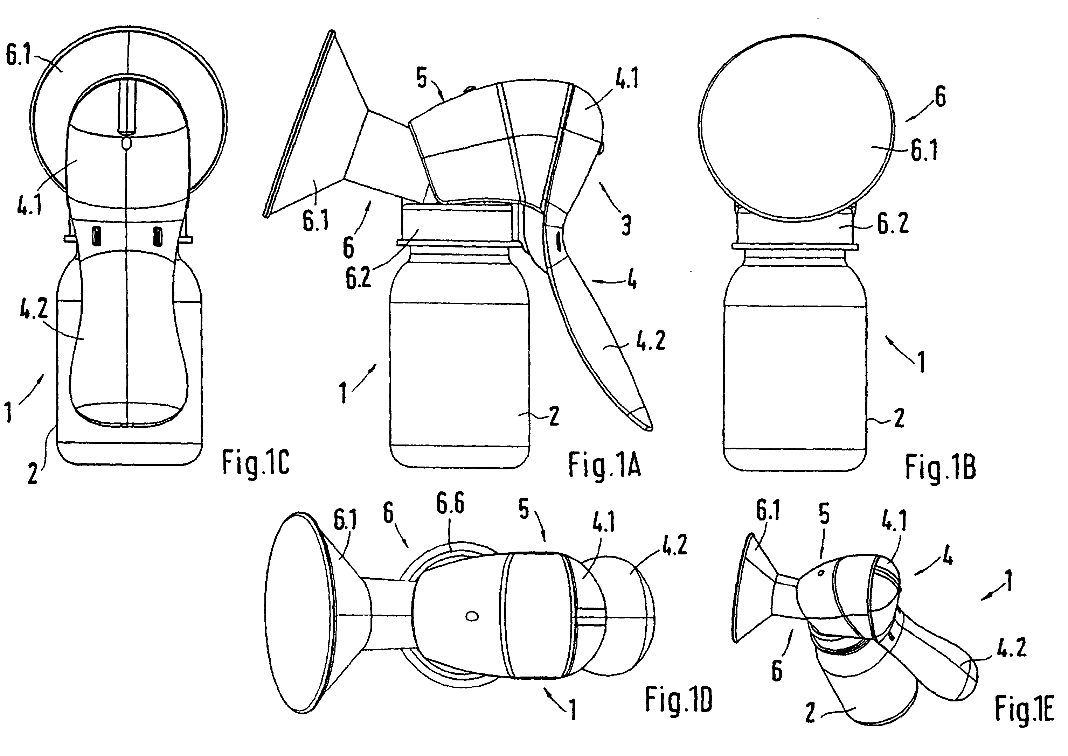

Wie aus Fig. 1 ersichtlich, weist die Milchabsaugpumpe 1 einen Behälter 2 zum Aufnehmen der abgesaugten Milch, ein auf diesem aufgeschraubtes Aufsatzteil 6, eine an diesem abnehmbar angebrachte Handpumpeneinheit 3 aus einem Betätigungsgriff 4 und mit diesem abnehmbar verbundenen Kappenteil 5 auf.

Das Aufsatzteil 6 ist mit einem Schraubanschluss 6.2 an einem eine Behälteröffnung umgebenden Halsabschnitt des Behälters 2 aufgeschraubt. Auf einem Deckelabschnitt des Schraubanschlusses 6.2 ist ein Brust-Ansatzstück 6.1 in an sich bekannter Weise angeschlossen, wobei etwa im Bereich des Deckelteils ein Durchlassventil angeordnet ist, durch das die abgesaugte Milch in den Behälter 2 gelangt und an einem Austreten aus dem Behälter 2 gehindert ist bzw. nicht mehr zurückgesaugt werden kann.

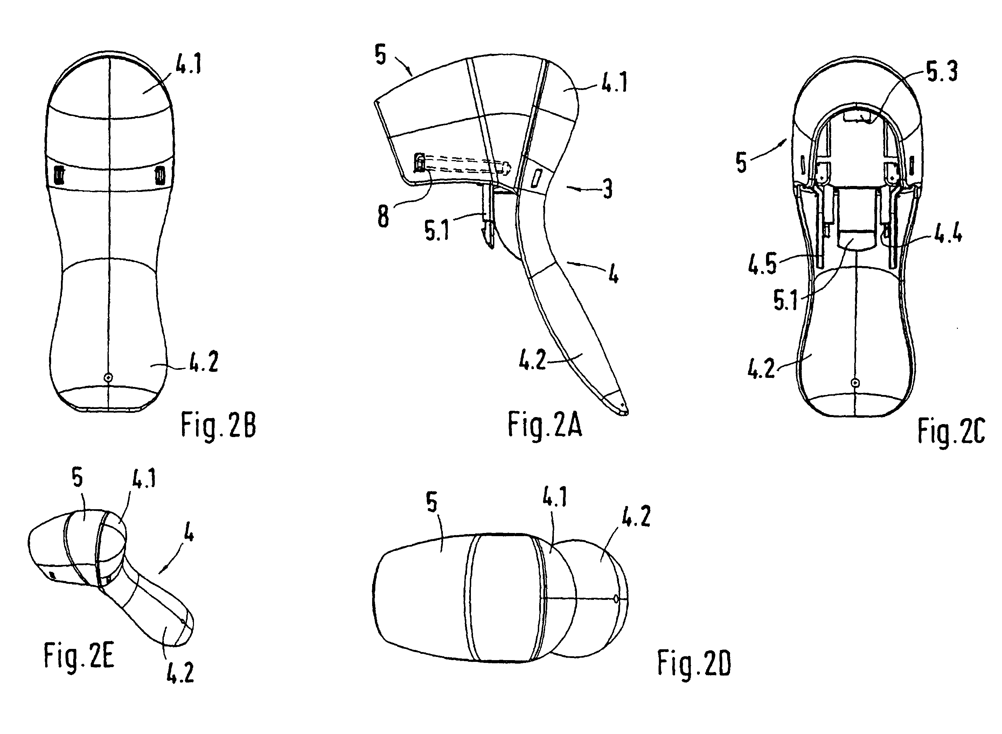

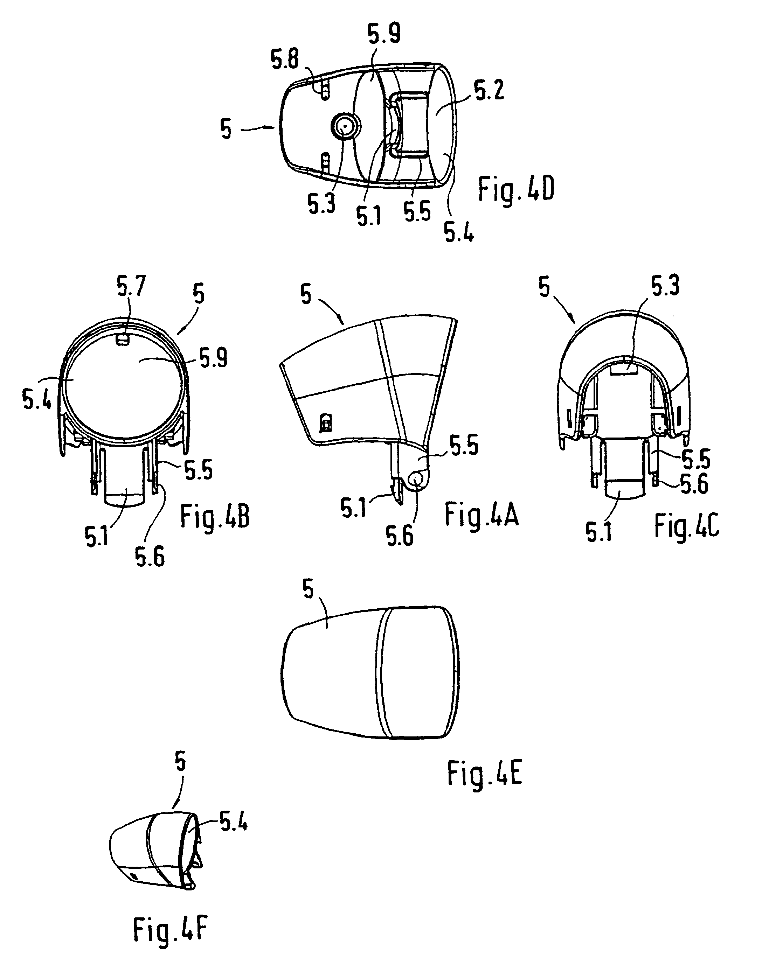

Auf dem Aufsatzteil 6 ist die Handpumpeneinheit 3 mit dem Kappenteil 5 auf ein parallel zur Behälterachse nach oben ragendes stutzenförmiges Anschlussstück mit einem entsprechend angepassten und z.B. durch eine Konusabdichtung oder ein zusätzliches Dichtungselement abgedichteten Koppelstück 5.3 dicht aufgesetzt, das in den Fig. 2C, 4C und 4D dargestellt ist. Das Koppelstück 5.3 ist auf der Innenseite der oberen Wandung des Kappenteils 5 nach unten stehend angeformt und geht in einen Verbindungskanal 5.7 über, der in einem ebenfalls in dem Kappenteil 5 ausgebildeten Hubraum mündet, wie Fig. 4B zeigt. Auf der Unterseite des Kappenteils 5 ragt ein Haltestück 5.1 in Form einer Rastzunge vor, die mit einem Rastabsatz am unteren Rand des Schraubanschlusses 6.2 einrastet, wobei die Rastzunge mittels einer endseitigen Rastschräge beim Aufsetzen des Kappenteils 5 gegen ihre Federkraft ausgelenkt wird.

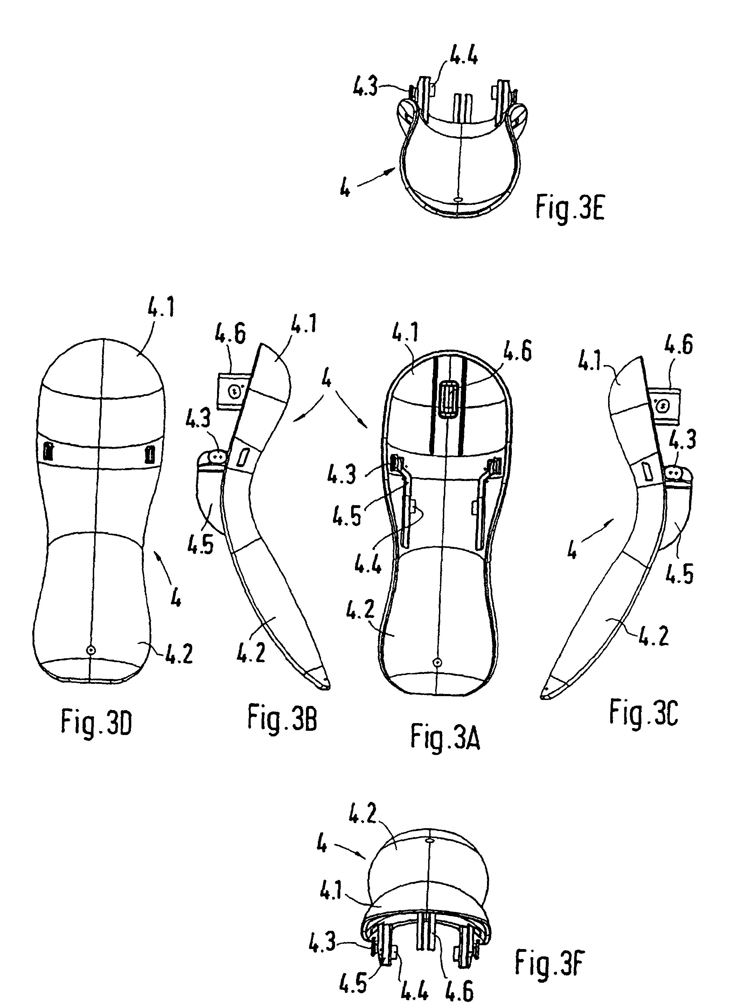

Der Hubraum 5.2 ist in dem von dem Ansatzstück 6.1 abgekehrten hinteren Abschnitt des Kappenteils 5 angeordnet und besitzt auf seiner Rückseite eine Hubraumöffnung 5.4, während er nach vorne mittels einer Stirnwand 5.9 abgeschlossen ist. Der Hubraum 5.2 ist entsprechend einer Bewegungsbahn eines darin mittels des Betätigungsgriffes 4 hin und her bewegten Pumpkolbens 7 gekrümmt ausgebildet. An der Unterseite des Kappenteils 5 sind seitlich Haltelappen bzw. Haltestege 5.5 eines im Querschnitt U-förmigen Halteelementes vorgesehen, die in ihrem unteren Bereich in der Nähe des freien Endes Lageraugen 5.6 zum Einsetzen entsprechend angepasster Lagerzapfen 4.4 des Betätigungsgriffes 4 aufweisen, um eine abnehmbare, gelenkige Anbindung des Betätigungsgriffes 4 an dem Kappenteil 5 herzustellen. An der Innenseite des vorderen Abschnittes des Kappenteils 5 sind desweiteren auf beiden Seiten im Wesentlichen parallel zur Behälterachse verlaufende Stege angeformt, sowie Einhängeelemente 5.8, in die Rückholfedern 8, z.B. Gummiringe, eingehängt werden, um den an dem Kappenteil 5 angebrachten Betätigungsgriff 4 nach einem Auslenken wieder in die Ausgangsstellung, in der der Pumpkolben 7 in den Hubraum 5.2 eingeführt ist, mittels Federkraft zurückzuführen, wie aus Fig. 2A ersichtlich. Entsprechende weitere Einhängeelemente 4.3 sind an Stegen 4.5 auf der Innenseite des Betätigungsgriffes 4 angebracht. Die Stege 4.5 tragen auch die Lagerzapfen 4.4, wie aus den Fig. 2C, 3A, 3E und 3F ersichtlich.

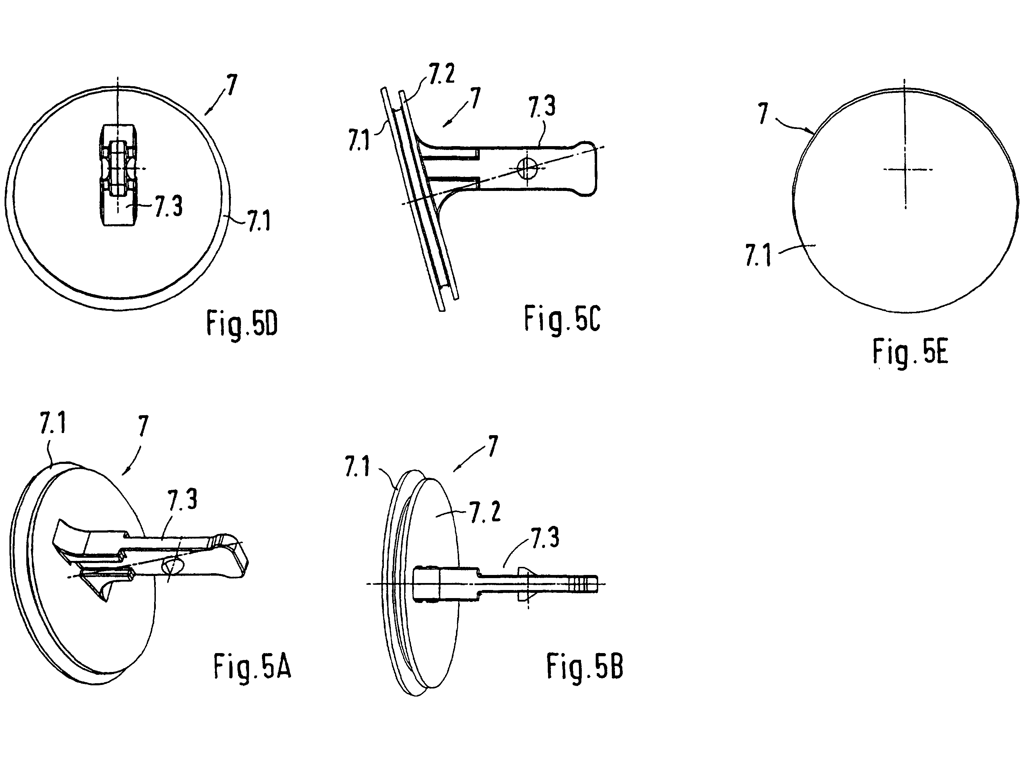

Der Betätigungsgriff 4 geht mit seinem oberhalb der Gelenkachse liegenden oberen Abschnitt 4.1 in stetiger Krümmung oben und seitlich in die Rundungen des Kappenteils 5 über und verschließt die Hubraumöffnung 5.4 in der vollständig eingeschobenen Stellung des Pumpkolbens 7, wie die Fig. 1 A und 2A zeigen. An den oberen Abschnitt 4.1 des Betätigungsgriffes 4 schließt sich etwa im Bereich der Schwenkachse ein stumpfwinklig nach hinten gerichteter unterer Abschnitt 4.2 an, der im Querschnitt ebenfalls konvex nach außen gekrümmt ist, so dass sich eine gute Handhabung des unteren Griffabschnittes 4.2 zum Ausführen einer Pumpbewegung ergibt, wobei der untere Abschnitt 4.2 am Handballen anliegt und im Übergangsbereich zwischen dem oberen Abschnitt 4.1 und dem unteren Abschnitt 4.2 Daumen und Zeigefinger nach vorne gerichtet den oberen Behälterabschnitt erfassen können. Auf der Innenseite des oberen Abschnittes 4.1 ist ein Verbindungsstück 4.6 angeformt, um eine Verbindung mit einer Kolbenstange 4.3 des Pumpkolbens 7 herzustellen. Die Kolbenstange 7.3 ist, wie aus den Fig. 5A bis 5D ersichtlich, an einer Halteplatte 7.2 angeformt, die ihrerseits Teil einer Kolbenplatte 7.1 mit seitlichen Dichträndern bildet. Im unteren Abschnitt 4.2 des Betätigungsgriffes kann auf der Innenseite ein nicht näher dargestelltes weiches Abstandselement vorgesehen sein, so dass der untere Abschnitt 4.2 beim Betätigung weich an die Außenseite des Behälters 2 anschlägt und auch eine Begrenzung des Hubweges des Pumpkolbens 7 damit einstellbar ist. Weiterhin kann zur Begrenzung des Hubweges und damit der Pumpwirkung ein ebenfalls nicht dargestelltes Zwischenstück vorgesehen sein, das beim Ausschwenken des oberen Abschnittes 4.1 des Betätigungsgriffes 4 zwischen dem Rand der Hubraumöffnung 5.4 und dem oberen Abschnitt 4.1 einsetzbar ist. Auf der Rückseite des Aufsatzteiles 6 kann eine wahlweise freigebbare Öffnung zur Handbelüftung bei Anschluss einer Elektropumpe an dem Anschlussstutzen vorgesehen sein, die mit einem Stopfen verschließbar ist.

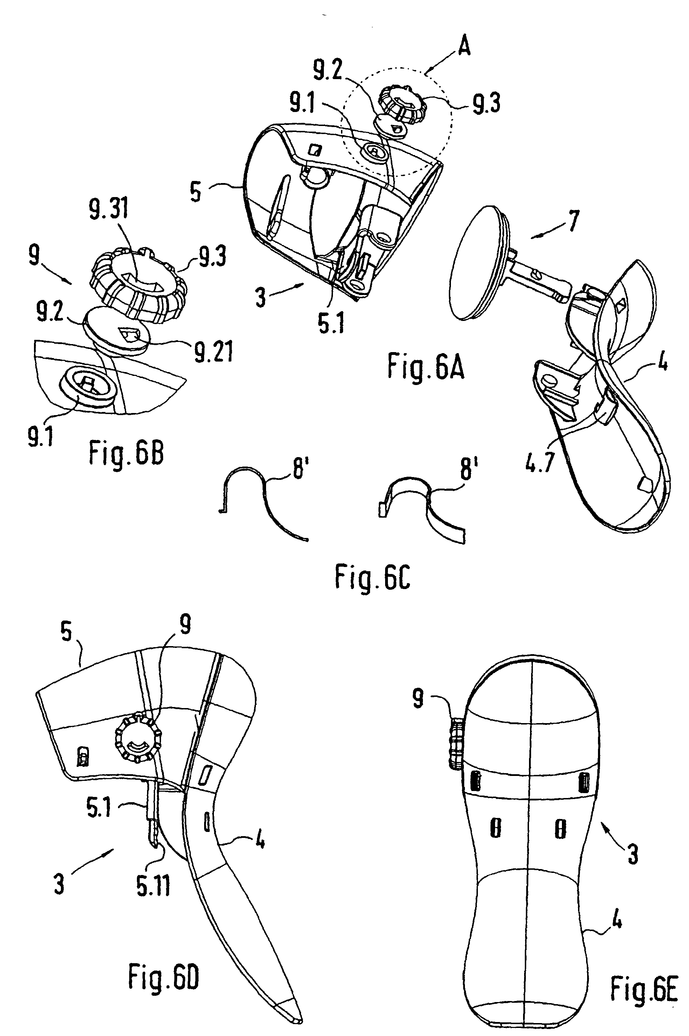

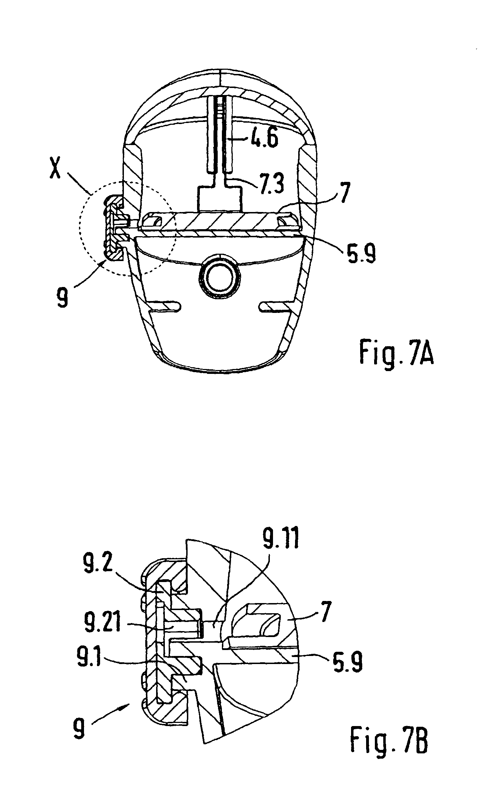

Die Fig. 6A bis 6E und 7A, 7B zeigen ein weiteres Ausführungsbeispiel der Milchabsaugpumpe 1, bei dem die Handpumpeneinheit 3 mit einer Beiluftreguliereinheit 9 und einem gegenüber den vorherigen Ausführungsbeispielen alternativen Rückholmechanismus 8' versehen ist. Im übrigen entspricht der Aufbau im Wesentlichen der vorstehend beschriebenen Ausführungsform, wobei sich die Bezugszeichen auf entsprechende Teile der Milchabsaugpumpe 1 beziehen.

Der in den Fig. 6A und 6C gezeigte Rückholmechanismus weist eine im Wesentlichen U-förmig gebogene Biegefeder 8' auf, wobei das Ende des einen, vorderen Schenkels zu einer Einhängenase abgewinkelt ist, während der andere Schenkel in einem nach außen gebogenen Endabschnitt ausläuft. Die Einhängenase wird in eine Aussparung im (in der Gebrauchsstellung) unteren Endabschnitt des Haltestückes 5.1 ausgebildete Aussparung in Form eines beim Fertigungsvorgang eingeformten Stützabschnittes 5.11 festgelegt, während das andere Schenkelende in ein an der Innenseite des Betätigungsgriffes 4 angeformtes Stützelement 4.7 eingesteckt und abgestützt ist. Die Biegefeder kann mit diesen Maßnahmen leicht montiert und leicht entfernt werden, z.B. beim Auseinandernehmen des Kappenteils 5 und des Betätigungsgriffes 4. Die Biegefeder ist vorzugsweise als Stahlfeder ausgebildet und behält ihre Federeigenschaften dauerhaft bei und kann auch problemlos durch Kochen gereinigt werden. Sie ragt im Einbauzustand mit der U-Wölbung nach oben in einen zwischen dem Kappenteil 5 und dem Betätigungsgriff 4 gebildeten Hohlraum, so dass sie optisch verdeckt untergebracht ist.

Wie aus den Fig. 6A, 6B, 6E, 6D und 7B, 7A ersichtlich, ist seitlich außen an der Wandung des Kappenteils 5 die Beiluftreguliereinheit 9 angeordnet. Sie weist einen in dem Kappenteil 5 ausgebildeten, nach außen etwas vorstehenden Einlassabschnitt 9.1 auf, in dem ein zentraler Aufnahmestift für einen von außen aufsetzbaren Einsatz 9.2 sowie ein Durchtrittskanal 9.11 vorgesehen sind, der in den Hubraum 5.2 in der Nähe der Stirnwand 5.9 mündet, wie aus den Fig. 7A und 7B ersichtlich. In dem Einsatz 9.2 ist eine mit dem Durchtrittskanal 9.11 zur Deckung gebrachte oder zur Deckung bringbare durchgehende Öffnung 9.21 ausgebildet. Der Einlassabschnitt 9.1 und der Einsatz 9.2 sind durch eine abnehmbare Abdeckung 9.3, vorzugsweise aus Silikon, abgedeckt, wobei ein Schlitz 9.31 mit der Öffnung 9.21 mehr oder weniger weit zur Deckung bringbar ist. Die Öffnung 9.21 mündet in einem seitlich verbreiterten Abschnitt, so dass das Ausmaß der Überdeckung des von dem Hubraum 5.2 nach außen führenden Kanals durch entsprechende Drehung der Abdeckung 9.3 in weiten Grenzen leicht variierbar ist. Die Abdeckung 9.3 weist zur einfachen Betätigung umfangsseitig rippenartig vorstehende Griffelemente auf, wobei eines als Markierung für die Drehstellung verdickt ist. Wie insbesondere aus Fig. 7B ersichtlich, untergreift ein lippenartiger Rand der Abdeckung 9.3 einen nach außen ragenden umlaufenden Bund des Einlassabschnittes 9.1, so dass sich ein guter Halt und eine gute Abdichtung für die Abdeckung 9.3 ergeben.

Die Beiluftreguliereinheit 9 ergibt eine stufenlose, reproduzierbare Reguliermöglichkeit für in den Saugraum der Milchabsaugpumpe geführte Beiluft. Ein aufgebautes Vakuum wird bei mehr oder weniger weit geöffneter Beiluftöffnung automatisch abgebaut, und die Höhe des Vakuums kann individuell gewählt werden. Es kann ein automatisches intermittierendes Abpumpen vorgenommen werden.

## Patentansprüche

1. Milchabsaugpumpe mit einem an der Öffnung eines Behälters (2) lösbar angebrachten oder anbringbaren, ein Brustansatzstück (6.1) aufweisenden Aufsatzteil (6) und einer an diesem mittels eines Anschlussstutzens (5.3) oder einer Anschlussbohrung lösbar angeschlossenen Handpumpeneinheit (3), die einen kappenartigen Anschlussabschnitt sowie einen in einem Hubraum (5.2) mittels eines schwenkbaren und mit einem Rückholmechanismus (8, 8') versehenen Betätigungsgriffes (4) hin und her bewegbaren Pumpkolben (7) aufweist,
**dadurch gekennzeichnet,**
**dass** der kappenartige Anschlussabschnitt und der Hubraum (5.2) in einem als einheitliches kappenförmiges Teil ausgebildeten Kappenteil (5) zusammengefasst sind, das mit Haltemitteln (5.1, 5.3) ausschließlich an dem Aufsatzteil (6) festgelegt ist, und
**dass** der einerseits an dem Betätigungsgriff (4) angreifende Rückholmechanismus (8) andererseits an dem Kappenteil (5) gelagert ist.

2. Milchabsaugpumpe nach Anspruch 1,
**dadurch gekennzeichnet,**
**dass** eine Hubraumöffnung (5.4) auf der dem Brustansatzstück (6.1) abgekehrten Seite des Hubraums (5.2) im vollkommen eingeführten Zustand des Pumpkolbens (7) mit einem in Gebrauchsstellung oberhalb einer Schwenkachse liegenden oberen Abschnitt (4.1) des Betätigungsgriffes (4) abgedeckt ist.

3. Milchabsaugpumpe nach Anspruch 1 oder 2,
**dadurch gekennzeichnet,**
**dass** der Hubraum (5.2) in dem Kappenteil (5) entsprechend einer Bewegungsbahn des von dem oberen Abschnitt (4.1) des Betätigungsgriffes (4) bewegten Pumpkolbens (7) bogenförmig gekrümmt ist.

4. Milchabsaugpumpe nach einem der vorhergehenden Ansprüche,
**dadurch gekennzeichnet,**
**dass** ein Schwenkweg des oberen Abschnittes (4.1) des Betätigungsgriffes (4) im Bereich seiner Anbindung an den Pumpkolben (7) bei angebrachtem Behälter so groß gewählt ist, dass zumindest ein oberer Randabschnitt des Pumpkolbens im herausbewegten Zustand außerhalb eines oberen Öffnungsrandes der Hubraumöffnung (5.4) liegt.

5. Milchabsaugpumpe nach einem der vorhergehenden Ansprüche,
**dadurch gekennzeichnet,**
**dass** der Rückholmechanismus (8) mindestens eine Zugfeder aufweist,
**dass** ein Einhängeelement (4.3) an dem Betätigungsgriff (4) und ein weiteres Einhängeelement an dem Kappenteil (5) derart positioniert sind, dass bei eingeführter Kolbenstellung die Richtung der Zugkraft oberhalb der Schwenkachse des Betätigungsgriffes (4) liegt, zumindest bis der Betätigungsgriff (4) bei angebrachtem Behälter (2) seinen maximalen Schwenkwinkel in Ausführrichtung des Pumpkolbens (7) erreicht, und
**dass** bei abgenommenem Behälter (2) und weiter vergrößertem Schwenkwinkel die Richtung der Zugkraft unterhalb die Schwenkachse tritt, so dass der Betätigungsgriff (4) gegenüber dem Kappenteil (5) in der geöffneten Stellung gehalten ist.

6. Milchabsaugpumpe nach einem der Ansprüche 1 bis 4,
**dadurch gekennzeichnet,**
**dass** der Rückholmechanismus (8') mindestens eine Druckfeder aufweist,
**dass** ein Stützelement (4.7) auf der Innenseite des Betätigungsgriffes (4) und ein Stützabschnitt (5.11 ) an dem Kappenteil (5) derart positioniert sind,
**dass** zumindest bei eingeführter Kolbenstange die Richtung der Druckkraft unterhalb der Schwenkachse des Betätigungsgriffes (4) liegt.

7. Milchabsaugpumpe nach Anspruch 6,
**dadurch gekennzeichnet,**
**dass** die Druckfeder als Biegefeder ausgebildet ist, die mit einer vorderen Einhängenase an einem Stützabschnitt (5.11 ) in einem beim Gebrauch nach unten gerichteten freien Endabschnitt des Haltestückes (5.1) eingehängt und abgestützt ist, sich in einen Innenraum von Kappenteil (5) und Betätigungsgriff U-förmig nach oben aufwölbt und mit einem von der Einhängenase abgelegenen freien Endbereich an einem auf der Innenseite des Betätigungsgriffes (4) angeordneten Stützelement (4.7) abgestützt ist.

8. Milchabsaugpumpe nach einem der vorhergehenden Ansprüche,
**dadurch gekennzeichnet,**
**dass** an dem an dem Aufsatzteil (6) angeordneten Anschlussstutzen oder der Anschlussbohrung eine Elektropumpe unmittelbar mit einem Schlauch anschließbar ist.

9. Milchabsaugpumpe nach einem der vorhergehenden Ansprüche,
**dadurch gekennzeichnet,**
**dass** die Ankoppelstelle zwischen dem Anschlussstutzen (5.3) oder der Anschlussbohrung und dem Kappenteil (5) mittels einer Konusverbindung oder einem Dichtring abgedichtet ist.

10. Milchabsaugpumpe nach einem der vorhergehenden Ansprüche,
**dadurch gekennzeichnet,**
**dass** an dem Aufsatzteil (6) im Bereich des Anschlussstutzens (5.3) oder der Anschlussbohrung eine mittels eines Stopfens und, bei abgenommenem Stopfen, von Hand verschließbare Öffnung vorgesehen ist.

11. Milchabsaugpumpe nach einem der vorhergehenden Ansprüche,
**dadurch gekennzeichnet,**
**dass** die Haltemittel ein Rastelement (5.1) aufweisen, das bei an dem Aufsatzteil (6) angekoppeltem Kappenteil (5) an dem Aufsatzteil (6) verrastet.

12. Milchabsaugpumpe nach Anspruch 11,
**dadurch gekennzeichnet,**
**dass** das Rastelement als mit einem freien Endabschnitt zu dem Behälter (2) gerichtete Rastzunge (5.1) ausgebildet ist, die im aufgesetzten Zustand mit einem Rastabsatz einen zum Behälter (2) gekehrten Rand des Aufsatzteils (6) hintergreift, wenn das Kappenteil (5) der Rastzunge (5.1) gegenüberliegend auf den bezüglich des Behälters (2) achsparallel gerichteten Anschlussstutzen (5.3) oder die Anschlussbohrung aufgeschoben ist.

13. Milchabsaugpumpe nach einem der vorhergehenden Ansprüche,
**dadurch gekennzeichnet,**
**dass** der Kappenteil (5) auf seiner Innenseite beidseitig Haltelappen (5.5) oder Rippen trägt, die Führungselemente beim Aufsetzen auf das Aufsatzteil (6) und Sicherungselemente gegen ein Verdrehen des Kappenteils (5) bezüglich des Aufsatzteils (6) bilden.

14. Milchabsaugpumpe nach einem der vorhergehenden Ansprüche,
**dadurch gekennzeichnet,**
**dass** an beiden Seitenabschnitten des Kappenteils (5) einerseits und beiden Seitenabschnitten des Betätigungsgriffes (4) andererseits Lagerelemente angeordnet sind, die als trennbare Lagerzapfen/Lagerauge-Verbindung die Schwenkachse zwischen Betätigungsgriff (4) und Kappenteil (5) bilden.

15. Milchabsaugpumpe nach einem der Ansprüche 2 bis 14,
**dadurch gekennzeichnet,**
**dass** das Kappenteil (5) eine gerundete Oberseite aufweist, die in dem von dem Brustansatzstück (6.1) abgekehrten hinteren Bereich im eingeschwenkten Zustand des oberen Abschnittes (4.1) des Betätigungsgriffes (4) stetig in dessen ebenfalls gekrümmte Außenseite übergeht.

16. Milchabsaugpumpe nach einem der vorhergehenden Ansprüche,
**dadurch gekennzeichnet,**
**dass** der obere Abschnitt (4.1) und der untere Abschnitt (4.2) im Querschnitt auf ihrer von dem Brustansatzstück (6.1) abgekehrten Rückseite nach außen gerundet sind und stetig ineinander übergehen, wobei zwischen dem oberen Abschnitt (4.1) und dem unteren Abschnitt (4.2) ein nach hinten offener stumpfer Winkel gebildet ist.

17. Milchabsaugpumpe nach einem der vorhergehenden Ansprüche,
**dadurch gekennzeichnet,**
**dass** ein in den beim Verschwenken des Betätigungsgriffes (4) im oberen Bereich zwischen dessen oberem Abschnitt (4.1) und dem Rand der Hubraumöffnung (5.4) gebildeten V-förmigen Spalt einsetzbares Zwischenstück vorgesehen ist, mit dem der Hubweg des Pumpenkolbens (7) stufenlos oder abgestuft vorgebbar ist.

18. Milchabsaugpumpe nach einem der vorhergehenden Ansprüche,
**dadurch gekennzeichnet,**
**dass** auf der Innenseite eines Schraubanschlusses (6.2) des Aufsatzteils (6) zum Verbinden mit dem Behälter (2) Abstandsnocken vorgesehen sind, die im aufgesetzten Zustand mit dem oberen Rand des Behälters (2) in Berührung treten, so dass im aufgesetzten Zustand ein Luftaustausch mit der Atmosphäre gegeben ist.

19. Milchabsaugpumpe nach einem der vorhergehenden Ansprüche,
**dadurch gekennzeichnet,**
**dass** der Pumpkolben (7) zentrisch oder exzentrisch nach oben versetzt eine angeformte Kolbenstange (7.3) aufweist, an dessen nach hinten gerichtetem Endabschnitt eine lösbare, gelenkige Verbindung mit dem oberen Abschnitt (4.1) des Betätigungsgriffes (4) hergestellt ist.

20. Milchabsaugpumpe nach einem der vorhergehenden Ansprüche,
**dadurch gekennzeichnet,**
**dass** auf der behälterseitigen Innenseite des unteren Abschnittes (4.2) des Betätigungsgriffes (4) ein Vorsprung aus weichem Material zum Bilden eines Anschlages zwischen dem Betätigungsgriff (4) und dem Behälter (2) angeordnet ist.

21. Milchabsaugpumpe nach einem der vorhergehenden Ansprüche,
**dadurch gekennzeichnet,**
**dass** die Handpumpeneinheit (3) und das Aufsatzteil (6) so angeordnet und in ihrem Gewicht abgeglichen sind, dass der Behälter (2) im leeren Zustand bei aufgesetztem Aufsatzteil (6) und angebrachter Handpumpeneinheit (3) aufrecht stehen bleibt.

22. Milchabsaugpumpe nach einem der vorhergehenden Ansprüche,
**dadurch gekennzeichnet,**
**dass** an dem Kappenteil (5) eine von außen manuell betätigbare Beiluftreguliereinheit (9) zur Belüftung des bei einer Pumpenbetätigung variierten Saugraumes vorgesehen ist.

23. Milchabsaugpumpe nach Anspruch 22,
**dadurch gekennzeichnet,**
**dass** die Beiluftreguliereinheit (9) mit einem außen auf dem Kappenteil (5) angeordneten, drehbaren Einsatzteil (9.2) und/oder Aufsatzteil (9.3) versehen ist, mit dessen oder deren Verdrehung ein durch eine Wandung des Kappenteils (5) in den Hubraum (5.2) führender Durchtrittskanal mehr oder weniger weit geöffnet wird oder ganz verschließbar ist.

## Claims

1. Milk extraction pump, having an attachment (6), which is detachably mounted or mountable on the opening of a container (2) and has a breast extension-piece (6.1), and a hand pump unit (3), which detachably communicates with said attachment by means of a connection-piece (5.3) or a connecting bore and has a cap-like connection portion as well as a pump piston (7), which is reciprocatable in a displacement area (5.2) by means of a pivotable actuating handle (4) provided with a return mechanism (8, 8'), **characterised in that** the cap-like connection portion and the displacement area (5.2) are combined in a cap part (5), which is in the form of a uniform cap-shaped part and is exclusively secured on the attachment (6) by retaining means (5.1, 5.3), and **in that** the return mechanism (8), which co-operates with the actuating handle (4) at one end, is mounted on the cap part (5) at the other end.

2. Milk extraction pump according to claim 1, **characterised in that**, when the pump piston (7) is in the completely inserted state, a displacement area aperture (5.4) is covered with an upper portion (4.1) of the actuating handle (4), situated above a pivotal axis in the position of use, on the side of the displacement area (5.2) remote from the breast extension-piece (6.1).

3. Milk extraction pump according to claim 1 or 2, **characterised in that** the displacement area (5.2) in the cap part (5) is curved in an arcuate manner along a displacement path of the pump piston (7) displaced by the upper portion (4.1) of the actuating handle (4). preceding claims,

4. Milk extraction pump according to one of the preceding claims, **characterised in that**, when the container is mounted, a pivotal path of the upper portion (4.1) of the actuating handle (4), in the region of its connection with the pump piston (7), is selected to be so long that, in the outwardly displaced state, at least one upper edge portion of the pump piston is situated externally of an upper edge of the displacement area aperture (5.4).

5. Milk extraction pump according to one of the preceding claims, **characterised in that** the return mechanism (8) has at least one tension spring, **in that** a suspension element (4.3) on the actuating handle (4) and an additional suspension element on the cap part (5) are positioned in such a manner that, when the piston is in the inserted position, the direction of the tensile force lies above the pivotal axis of the actuating handle (4), at least until the actuating handle (4) reaches its maximum pivotal angle in the outward direction of the pump piston (7) when the container (2) is mounted, and **in that**, when the container (2) is removed, and when the pivotal angle is further increased, the direction of the tensile force lies beneath the pivotal axis, so that the actuating handle (4) is retained in the opened position relative to the cap part (5).

6. Milk extraction pump according to one of claims 1 to 4, **characterised in that** the return mechanism (8') has at least one compression spring, **in that** a supporting element (4.7) on the inside of the actuating handle (4) and a supporting portion (5.11 ) on the cap part (5) are positioned in such a manner that, at least when the piston rod is inserted, the direction of the compressive force lies beneath the pivotal axis of the actuating handle (4).

7. Milk extraction pump according to claim 6, **characterised in that** the compression spring is in the form of a bending spring, which is suspended and supported by a front suspension projection on a supporting portion (5.11 ) in a free end portion of the retaining piece (5.1), which end portion is orientated downwardly during use, said spring curving upwardly in a U-shaped manner in an interior of the cap part (5) and actuating handle (4) and being supported by a free end region, remote from the suspension projection, on a supporting element (4.7) disposed on the inside of the actuating handle (4).

8. Milk extraction pump according to one of the preceding claims, **characterised in that** an electric pump can communicate directly with the connection-piece, disposed on the attachment (6), or with the connecting bore by means of a hose.

9. Milk extraction pump according to one of the preceding claims, **characterised in that** the coupling location between the connection-piece (5.3) or the connecting bore and the cap part (5) is sealed by means of a conical connection or a sealing ring.

10. Milk extraction pump according to one of the preceding claims, **characterised in that** an opening, which can be closed by means of a stopper and by hand if the stopper has been removed, is provided on the attachment (6) in the region of the connection-piece (5.3) or the connecting bore.

11. Milk extraction pump according to one of the preceding claims, **characterised in that** the retaining means have a locking element (5.1), which engages on the attachment (6) when the cap part (5) is connected to the attachment (6).

12. Milk extraction pump according to claim 11, **characterised in that** the locking element is in the form of a locking projection (5.1), which is orientated towards the container (2) with a free end portion and, in the mounted state, engages behind an edge of the attachment facing the container (2) with a locking shoulder when the cap part (5), situated opposite the locking projection (5.1), is slipped over the connection-piece (5.3), which is orientated in an axis-parallel manner relative to the container (2), or over the connecting bore.

13. Milk extraction pump according to one of the preceding claims, **characterised in that** the cap part (5) has, on its inside, at both ends, retaining lugs (5.5) or ribs which form guide elements for mounting on the attachment (6) and securing elements to prevent the cap part (5) from twisting relative to the attachment (6).

14. Milk extraction pump according to one of the preceding claims, **characterised in that** bearing elements are disposed on both lateral portions of the cap part (5) at one end and on both lateral portions of the actuating handle (4) at the other end, said bearing elements forming, as a separable bearing pin/bearing eyelet connection, the pivotal axis between the actuating handle (4) and cap part (5).

15. Milk extraction pump according to one of claims 2 to 14, **characterised in that** the cap part (5) has a rounded upper surface which, in the rear region remote from the breast extension-piece (6.1), constantly passes into an equally curved external surface of the actuating handle (4) when the upper portion (4.1) of said handle is in the inwardly pivoted state.

16. Milk extraction pump according to one of the preceding claims, **characterised in that** the upper portion (4.1 ) and the lower portion (4.2) are outwardly rounded in cross-section on their rear surface remote from the breast extension-piece (6.1) and constantly pass into each other, a rearwardly open obtuse angle being formed between the upper portion (4.1) and the lower portion (4.2).

17. Milk extraction pump according to one of the preceding claims, **characterised in that** an intermediate piece is provided, which is insertable into the V-shaped gap formed, during the pivotal movement of the actuating handle (4), between the upper portion (4.1) of said handle and the edge of the displacement area aperture (5.4), the displacement path of the pump piston (7) being prescribable by said intermediate piece in a stepless or stepped manner.

18. Milk extraction pump according to one of the preceding claims, **characterised in that** spacer cams are provided on the inside of a screw connection (6.2) of the attachment (6) for connection with the container (2), said cams coming into contact with the upper edge of the container (2) in the attached state, so that there is an exchange of air with the atmosphere in the attached state.

19. Milk extraction pump according to one of the preceding claims, **characterised in that** the pump piston (7) has a moulded piston rod (7.3) centrally or excentrically in an upwardly offset manner, a detachable pivotal connection being established with the upper portion (4.1) of the actuating handle (4) at the rearwardly orientated end portion of said piston.

20. Milk extraction pump according to one of the preceding claims, **characterised in that** a projection member, formed from soft material, is disposed on the inside of the lower portion (4.2) of the actuating handle (4) at the container end for forming a stop member between the actuating handle (4) and the container (2).

21. Milk extraction pump according to one of the preceding claims, **characterised in that** the hand pump unit (3) and the attachment (6) are so disposed and are so adapted in respect of their weight that the container (2) remains upright in the empty state when the attachment (6) is attached and the hand pump unit (3) is mounted.

22. Milk extraction pump according to one of the preceding claims, **characterised in that** a secondary air regulating unit (9), which is manually actuatable from externally, is provided on the cap part (5) to ventilate the suction chamber which has altered during a pump actuation.

23. Milk extraction pump according to claim 22, **characterised in that** the secondary air regulating unit (9) is provided with a rotatable insert (9.2), disposed externally on the cap part (5), and/or with an attachment (9.3), by means of which, or by means of the rotation of said unit, a through-passage, leading through a wall of the cap part (5) into the displacement area (5.2), is opened more or less widely or is completely closable.

## Revendications

1. Tire-lait, avec un élément de couronnement (6) monté ou pouvant être monté de manière amovible sur l'ouverture d'un récipient (2) et présentant une pièce (6.1) d'application contre la poitrine, et avec un ensemble de pompe à main (3) raccordé de manière amovible à l'élément de couronnement (6) au moyen d'une tubulure de raccordement (5.3) ou d'un perçage de raccordement, ensemble qui présente une partie de raccordement du genre calotte et un piston de pompe (7) mobile en va-et-vient dans une chambre de piston (5.2) au moyen d'une poignée d'actionnement pivotante (4), pourvue d'un mécanisme de rappel (8, 8'),
**caractérisé en ce que** la partie de raccordement du genre calotte et la chambre de piston (5.2) sont regroupées en un élément formant calotte (5), conçu comme élément homogène en forme de calotte, qui est fixé exclusivement sur l'élément de couronnement (6) par des moyens de maintien (5.1, 5.3),
et **en ce que** le mécanisme de rappel (8), agissant d'un côté sur la poignée d'actionnement (4), est monté de l'autre côté sur l'élément formant calotte (5).

2. Tire-lait selon la revendication 1, **caractérisé en ce qu'**une ouverture (5.4) de chambre de piston, sur le côté de la chambre de piston (5.2) qui est opposé à la pièce (6.1 ) d'application contre la poitrine, est recouverte, lorsque le piston de pompe (7) est totalement rentré, par une partie supérieure (4.1), située en position d'utilisation au-dessus d'un axe de pivotement, de la poignée d'actionnement (4).

3. Tire-lait selon la revendication 1 ou 2, **caractérisé en ce que** la chambre de piston (5.2) dans l'élément formant calotte (5) est courbée en arc conformément à une trajectoire de déplacement du piston de pompe (7) déplacé par la partie supérieure (4.1) de la poignée d'actionnement (4).

4. Tire-lait selon l'une des revendications précédentes, **caractérisé en ce qu'**une course de pivotement de la partie supérieure (4.1) de la poignée d'actionnement (4) dans la région de son rattachement au piston de pompe (7), le récipient étant monté, est choisie suffisamment grande pour qu'au moins une partie de bord supérieur du piston de pompe à l'état sorti se trouve au-dessus d'un bord supérieur de l'ouverture (5.4) de chambre de piston.

5. Tire-lait selon l'une des revendications précédentes, **caractérisé**
**en ce que** le mécanisme de rappel (8) présente au moins un ressort de traction,
**en ce qu'**un élément d'accrochage (4.3) sur la poignée d'actionnement (4) et un autre élément d'accrochage sur l'élément formant calotte (5) sont positionnés de telle sorte que, dans la position rentrée du piston, la direction de la force de traction se trouve au-dessus de l'axe de pivotement de la poignée d'actionnement (4) au moins jusqu'à ce que la poignée d'actionnement (4) atteigne son angle maximal de pivotement, avec le récipient (2) monté, dans la direction de sortie du piston de pompe (7),
et **en ce que**, le récipient (2) étant démonté et l'angle de pivotement encore augmenté, la direction de la force de traction passe en dessous de l'axe de pivotement, de sorte que la poignée d'actionnement (4) est maintenue dans l'état ouvert par rapport à l'élément formant calotte (5).

6. Tire-lait selon l'une des revendications 1 à 4, **caractérisé**
**en ce que** le mécanisme de rappel (8') présente au moins un ressort de pression,
et **en ce qu'**un élément de soutien (4.7) sur le côté intérieur de la poignée d'actionnement (4) et une partie de soutien (5.11 ) sur l'élément formant calotte (5) sont positionnés de telle sorte, au moins lorsque la tige de piston est rentrée, la direction de la force de pression se situe en dessous de l'axe de pivotement de la poignée d'actionnement (4).

7. Tire-lait selon la revendication 6, **caractérisé en ce que** le ressort de pression est réalisé sous forme de ressort de flexion qui, par un bec d'accrochage avant, est accroché et soutenu dans une partie terminale libre, dirigée vers le bas en utilisation, de l'élément de maintien (5.1), s'incurve en U vers le haut dans un espace intérieur de l'élément formant calotte (5) et de la poignée d'actionnement (4), et s'appuie, par une région terminale libre opposée au bec d'accrochage, contre un élément de soutien (4.7) disposé sur le côté intérieur de la poignée d'actionnement (4).

8. Tire-lait selon l'une des revendications précédentes, **caractérisé en ce qu'**une pompe électrique peut, par un tuyau souple, être directement raccordée à la tubulure de raccordement ou au perçage de raccordement disposé(e) sur l'élément de couronnement (6).

9. Tire-lait selon l'une des revendications précédentes, **caractérisé en ce que** le point d'accouplement entre la tubulure de raccordement (5.3) ou le perçage de raccordement et l'élément formant calotte (5) est étanché au moyen d'un assemblage conique ou d'une bague d'étanchéité.

10. Tire-lait selon l'une des revendications précédentes, **caractérisé en ce qu'**une ouverture, pouvant être fermée par un bouchon et à la main lorsque le bouchon est retiré, est prévue sur l'élément de couronnement (6) dans la région de la tubulure de raccordement (5.3) ou du perçage de raccordement.

11. Tire-lait selon l'une des revendications précédentes, **caractérisé en ce que** les moyens de maintien présentent un élément d'enclenchement (5.1), qui s'enclenche sur l'élément de couronnement (6) lorsque l'élément formant calotte (5) est accouplé à l'élément de couronnement (6).

12. Tire-lait selon la revendication 11, **caractérisé en ce que** l'élément d'enclenchement est réalisé sous la forme d'une languette d'enclenchement (5.1), qui est dirigée vers le récipient (2) par une partie terminale libre et qui, dans l'état monté, s'engage par un décrochement d'enclenchement derrière un bord de l'élément de couronnement (6) qui est tourné vers le récipient (2), lorsque l'élément formant calotte (5) est enfoncé, en vis-à-vis de la languette d'enclenchement (5.1), sur la tubulure de raccordement (5.3) ou le perçage de raccordement orienté(e) parallèlement à l'axe du récipient (2).

13. Tire-lait selon l'une des revendications précédentes, **caractérisé en ce que** l'élément formant calotte (5) porte sur son côté intérieur, de part et d'autre, des pattes de maintien (5.5) ou des nervures, qui forment des éléments de guidage lors du montage sur l'élément de couronnement (6) et des éléments de blocage pour empêcher la rotation de l'élément formant calotte (5) par rapport à l'élément de couronnement (6).

14. Tire-lait selon l'une des revendications précédentes, **caractérisé en ce que** des éléments formant palier sont disposés sur les deux parties latérales de l'élément formant calotte (5) d'une part et sur les deux parties latérales de la poignée d'actionnement (4) d'autre part, éléments qui; en tant qu'assemblage détachable à pivot et bossage de palier, forment l'axe de pivotement entre la poignée d'actionnement (4) et l'élément formant calotte (5).

15. Tire-lait selon l'une des revendications 2 à 14, **caractérisé en ce que** l'élément formant calotte (5) présente une face supérieure arrondie qui, dans la région arrière opposée à la pièce (6.1) d'application contre la poitrine, dans l'état rentré par pivotement de la partie supérieure (4.1) de la poignée d'actionnement (4), se raccorde de façon continue à la face extérieure également courbe de celle-ci.

16. Tire-lait selon l'une des revendications précédentes, **caractérisé en ce que** la partie supérieure (4.1) et la partie inférieure (4.2) sont, en coupe transversale, sur leur face arrière opposée à la pièce (6.1) d'application contre la poitrine, arrondies vers l'extérieur et se raccordent de façon continue l'une dans l'autre, un angle obtus ouvert vers l'arrière étant formé entre la partie supérieure (4.1) et la partie inférieure (4.2).

17. Tire-lait selon l'une des revendications précédentes, **caractérisé en ce qu'**il est prévu une pièce intermédiaire, pouvant être insérée dans la fente en V formée lors du pivotement de la poignée d'actionnement (4) dans la région supérieure entre la partie supérieure (4.1) de celle-ci et le bord de l'ouverture (5.4) de chambre de piston, pièce qui permet de prescrire en continu ou par paliers la course du piston de pompe (7).

18. Tire-lait selon l'une des revendications précédentes, **caractérisé en ce que** des ergots d'écartement sont prévus sur la face intérieure d'un raccordement vissé (6.2) de l'élément de couronnement (6) pour l'assemblage avec le récipient (2), ergots qui, dans l'état monté, entrent en contact avec le bord supérieur du récipient (2), de sorte qu'on obtient dans l'état monté un échange d'air avec l'atmosphère.

19. Tire-lait selon l'une des revendications précédentes, **caractérisé en ce que** le piston de pompe (7) présente, décalée vers le haut de façon centrée ou excentrée, une tige de piston rapportée (7.3) sur la partie terminale, dirigée vers l'arrière, de laquelle est réalisé un assemblage articulé amovible avec la partie supérieure (4.1 ) de la poignée d'actionnement (4).

20. Tire-lait selon l'une des revendications précédentes, **caractérisé en ce qu'**une saillie en matériau souple, destinée à former une butée entre la poignée d'actionnement (4) et le récipient (2), est disposée sur la face intérieure, côté récipient, de la partie inférieure (4.2) de la poignée d'actionnement (4).

21. Tire-lait selon l'une des revendications précédentes, **caractérisé en ce que** l'ensemble de pompe à main (3) et l'élément de couronnement (6) sont disposés et réalisés avec des poids mutuellement adaptés de telle sorte que le récipient (2), à l'état vide, reste debout lorsque l'élément de couronnement (6) est monté et l'ensemble de pompe à main (3) installé.

22. Tire-lait selon l'une des revendications précédentes, **caractérisé en ce qu'**il est prévu sur l'élément formant calotte (5) une unité (9) de régulation d'air d'appoint, pouvant être actionnée manuellement de l'extérieur et destinée à ventiler la chambre d'aspiration variant lors d'un actionnement de la pompe.

23. Tire-lait selon la revendication 22, **caractérisé en ce que** l'unité (9) de régulation d'air d'appoint est pourvue d'une pièce d'insertion (9.2) et/ou de couronnement (9.3) rotative, disposée extérieurement sur l'élément formant calotte (5), dont la ou les rotation(s) permet(tent) d'ouvrir plus ou moins, ou de fermer totalement, un canal de passage menant dans la chambre de piston (5.2) à travers une paroi de l'élément formant calotte (5).
